# EUROPÄISCHE PATENTSCHRIFT

(11) **EP 3 449 830 B1**
(45) Veröffentlichungstag und Bekanntmachung des Hinweises auf die Patenterteilung: **29.01.2020**
(21) Anmeldenummer: 17188840.7
(22) Anmeldetag: 31.08.2017
(51) Int. Cl.: A61B 6/00, A61B 6/03

(54) **STEUERUNG EINES MEDIZINTECHNISCHEN BILDGEBENDEN SYSTEMS**
CONTROL OF A MEDICAL IMAGING DEVICE
COMMANDE D'UN SYSTÈME TECHNIQUE MÉDICALE DE CAPTEUR D'IMAGES

(43) Veröffentlichungstag der Anmeldung: 06.03.2019
(73) Patentinhaber: Siemens Healthcare GmbH, 91052 Erlangen (DE)
(72) Erfinder: Sühling, Michael, 91052 Erlangen (DE); Wimmer, Andreas, 91301 Forchheim (DE); Petry, Christian, 91094 Langensendelbach (DE)

(56) Entgegenhaltungen:
- DE-A1-102006 001 850
- DE-A1-102013 200 135
- DE-A1-102014 216 718
- US-A- 5 539 798

## Beschreibung

Die Erfindung betrifft ein Verfahren und eine Steuereinrichtung zur Steuerung eines medizintechnischen bildgebenden Systems sowie ein medizintechnisches bildgebendes System mit einer solchen Steuereinrichtung.

Medizinische Bildgebung wie Computertomographie oder Magnetresonanztomographie wie z.B. aus der DE 10 2006 001 850 A1 bekannt wird routinemäßig zur Diagnose von Krankheiten verwendet, insbesondere zur Behandlung oder zur Überwachung des Fortschritts der Therapie. Dabei werden Patienten in der Regel mehrmals gescannt. Zum Beispiel wird nach einer Krebsdiagnose ein Patient mehrmals im Laufe der Therapie medizinische Aufnahmen erstellt (z.B. mit einem Computertomographen "CT"), um den Verlauf der Krankheit zu überwachen. Zur Vorbereitung einer medizinischen Bildaufnahme ist es wichtig, den Ort und das Ausmaß des zu untersuchenden internen Bereichs zu kennen, z.B. um eine geeignete Positionierung bzw. eine Pose des Patienten auf dem Tisch auszuwählen.
Um eine medizinische Bildaufnahme vorzubereiten, werden oftmals externe Körpermerkmale verwendet, um die Lage der internen Strukturen grob zu erraten. Laser, z.B. an der Gantry eines CT werden eingesetzt, um die ausgewählte Region mit dem Scanner-Koordinatensystem abzugleichen. Die Tischhöhe wird gewöhnlich so gewählt, dass die Mitte der Körperoberfläche des Patienten mit dem Isozentrum der Gantry ausgerichtet ist. Planungsergebnisse werden zuweilen mit einem Stift auf den Patienten übertragen, indem man Markierungen auf die Haut zeichnet, zum Beispiel Einführungspunkte für Nadeln. Messwerkzeuge wie Dreieckslineale werden in der Regel verwendet, um einzuführende medizinische Geräte grob zu orientieren. Derzeit ist die Übertragung dieser Informationen an den Patienten, der auf dem Tisch des Scanners liegt, sehr schwierig.

All dies ist zeitintensiv und sehr ungenau, da es ohne die genaue Kenntnis der aktuellen Lage der realen inneren Organe geschieht, was einen großen Nachteil darstellt.

Es ist eine Aufgabe der vorliegenden Erfindung, ein Verfahren und eine Steuereinrichtung zur Steuerung eines medizintechnischen bildgebenden Systems zur Verfügung zu stellen, mit dem die oben beschriebenen Nachteile vermieden werden.

Diese Aufgabe wird durch ein Verfahren gemäß Patentanspruch 1, einer Steuereinrichtung nach Patentanspruch 10 sowie durch ein medizintechnisches bildgebendes System gemäß Patentanspruch 12 gelöst.

Das erfindungsgemäße Verfahren zur Steuerung eines medizintechnischen bildgebenden Systems bzw. zur Lokalisierung von inneren Strukturen in einem vorbestimmten Körperbereich, umfasst die folgenden Schritte.
- Bereitstellung eines dreidimensionalen Strukturbilds. Dieses dreidimensionalen Strukturbild liegt in der Regel in Form von dreidimensionalen Bilddaten vor und umfasst sowohl Oberflächendaten zu äußeren Konturen eines bestimmten Körperbereichs (auch als "Region of Interest" bezeichnet) sowie Strukturdaten zu inneren Strukturen dieses Körperbereichs. Die äußeren Konturen geben die Körperoberfläche des betreffenden Körperbereichs wieder. Die Strukturdaten bilden inneren Strukturen ab, z.B. Organe, Knochen oder Gefäße.

Ein Teil der Strukturdaten kann bevorzugt als interessierende Strukturen markiert sein oder markiert werden, z.B. nach der Registrierung. Als interessierende Strukturen werden diejenigen betrachtet, die bevorzugt durch das bildgebende medizintechnische System abgebildet werden sollen, z.B. da sie für die aktuell anstehende Aufnahme bzw. klinische Bewertung von besonderem Interesse sind.

Ein Teil der Strukturdaten kann aber auch bevorzugt als nichtinteressierende Strukturen markiert sein oder markiert werden, z.B. nach der Registrierung. Als nichtinteressierende Strukturen werden diejenigen betrachtet, die nicht durch das bildgebende medizintechnische System abgebildet werden sollen, z.B. da sie für die aktuell anstehende Aufnahme bzw. klinische Bewertung nicht von Interesse sind und daher zur Reduzierung der (Gesamt-) Strahlenbelastung nicht bestrahlt werden sollten.
- Aufnahme (zumindest) des Körperbereichs eines Patienten. Der Patient kann z.B. ein Mensch oder ein Tier sein. Die Aufnahme wird mittels eines Kamerasystems durchgeführt, während sich der Patient in dem bildgebenden medizinischen System befindet. Aus den Aufnahmen wird ein dreidimensionales Oberflächenbild zumindest des vorgenannten Körperbereichs des Patienten erstellt. Das Oberflächenbild bildet dabei einen Teil der Körperoberfläche ab, welche dem Körperbereich entspricht. Die Aufnahme umfasst dabei zumindest 3D-Daten, es ist aber bevorzugt, dass sie auch Informationen in Farben oder Grautönen zu der Körperoberfläche dieses Körperbereichs umfasst, wie unten genauer ausgeführt wird.
- Registrierung des Oberflächenbilds und des Strukturbilds.
Die Registrierung des Oberflächenbilds und des Strukturbilds aufeinander erfolgt zumindest bereichsweise. Die Registrierung kann z.B. mit Minimierung einer Kostenfunktion erreicht werden, welche die Abstände oder die Abstandsquadrate vorher festgelegter, miteinander übereinstimmender Punkte in den verschiedenen Bildern zueinander beinhaltet.

Da es zu dem Oberflächenbild in der Regel keine Daten zu den wahren inneren Strukturen des Patienten gibt, bedeutet die Registrierung des Oberflächenbildes mit dem Strukturbild, dass in der Praxis nur die Oberflächendaten des Strukturbildes für die Festlegung der notwendigen grafischen Transformationen für die Registrierung verwendet werden. Die inneren Strukturdaten des Strukturbildes werden jedoch entsprechend der mit der Registrierung einhergehenden Transformation der Oberflächendaten transformiert. Somit wird das Strukturbild mit dem Oberflächenbild also registriert, in dem die jeweiligen Oberflächen passend zusammengebracht werden und zumindest die oben genannten Strukturdaten im Verhältnis dazu angepasst werden bzw. in dem bei einer notwendigen grafischen Transformation das gesamte Strukturbild entsprechend transformiert wird.

Bei der Registrierung wird also bei einer Transformation des, vorzugsweise gesamten, Strukturbildes das Strukturbild passend auf das Oberflächenbild abgebildet, wobei die inneren Strukturen aufgrund der für die Registrierung gewählten Transformation mit einer statistischen Genauigkeit auf (nicht im Oberflächenbild enthaltene) entsprechende innere Strukturen des Patienten abgebildet würden.
- Erstellung eines Überlagerungsbilds.
   Das Überlagerungsbild umfasst dabei zumindest einen Teil des registrierten Strukturbilds inklusive registrierter Strukturdaten in Form einer Überlagerung mit einer Ansicht des Patienten. Dies kann das registrierte Oberflächenbild sein, insbesondere wenn wie weiter unten beschrieben eine VR-Anzeigevorrichtung verwendet wird, und/oder eine reale Ansicht des entsprechenden Teils des realen Körperbereichs sein, insbesondere wenn wie weiter unten beschrieben eine AR-Anzeigevorrichtung verwendet wird.
- Steuerung des medizintechnischen bildgebenden Systems. Das medizintechnische bildgebende System wird dabei dermaßen gesteuert, dass Bereiche des Patienten basierend auf der Anordnung der registrierten Strukturdaten im Überlagerungsbild aufgenommen werden. Bei der Steuerung sind dabei oftmals nicht nur Bereiche wichtig, sondern z.B. auch eine Schichtorientierung, ein Schichtabstand und/oder die Anzahl der Schichten. Auch eine Steuerung der Schichten kann also basierend auf dem Überlagerungsbild erfolgen, z.B. in dem für verschiedene Innere Strukturen vorgegebene oder automatisch erzeugte Aufnahmeparameter verwendet werden.

Bevorzugt wird das medizintechnische bildgebende System dabei so gesteuert, dass es Aufnahmen zumindest in demjenigen Bereich des Patienten macht, in dem im Überlagerungsbild diejenigen Strukturdaten angeordnet, die als interessierende Strukturen markiert sind und/oder, dass es keine Aufnahmen zumindest in demjenigen Bereich des Patienten macht, in dem im Überlagerungsbild diejenigen Strukturdaten angeordnet, die als nichtinteressierende Strukturen markiert sind. Damit findet in den Bereichen, die mit hoher Wahrscheinlichkeit den entsprechenden realen Strukturen entspricht, je nach Relevanz der Strukturdaten für die Untersuchung gezielt eine Bestrahlung oder gezielt keine Bestrahlung statt. Dadurch wird die durch die Aufnahmen zu erwartende Strahlenbelastung für den Patienten reduziert.

Eine diesbezügliche Steuerung ist mit Vorliegen des Überlagerungsbildes nicht schwer zu realisieren. Mit der dreidimensionalen Aufnahme des Körperbereichs des Patienten lässt sich das Oberflächenbild eindeutig in das Koordinatensystem des medizintechnischen bildgebenden Systems einordnen. Da das Strukturbild zusammen mit den Strukturdaten in dem Überlagerungsbild mit dem Oberflächenbild registriert ist, ist auch eine Positionierung basierend auf dem Koordinatensystem des medizintechnischen bildgebenden Systems in Abhängigkeit von den registrierten Strukturdaten möglich. Das medizintechnische bildgebende System muss also nur innerhalb seines Koordinatensystems gesteuert werden so dass bevorzugt gezielt eine Aufnahme für diejenigen Koordinaten erfolgt, an denen die registrierten interessierenden Strukturen liegen bzw. bevorzugt keine Aufnahme für diejenigen Koordinaten erfolgt, an denen die registrierten nichtinteressierenden Strukturen liegen.

Die erfindungsgemäße Steuereinrichtung zur Steuerung eines medizintechnischen bildgebenden Systems umfasst die nachfolgernd beschriebenen Komponenten.

Eine Strukturbildschnittstelle zur Bereitstellung eines dreidimensionalen Strukturbildes wie es oben bereits beschrieben wurde. Das Strukturbild umfasst sowohl Oberflächendaten zu äußeren Konturen sowie Strukturdaten zu inneren Strukturen eines Körperbereichs, wobei ein Teil der Strukturdaten bevorzugt die oben beschriebene Markierung als interessierende oder als nichtinteressierende Strukturen markiert ist.

Ein Kamerasystem zur Aufnahme zumindest des Körperbereichs des Patienten in einem bildgebenden medizinischen System. Ein solches Kamerasystem kann beispielsweise eine 3D-Kamera sein, z.B. ein Tiefensensor ggf. in Zusammenwirkung mit einer 2D-Kamera. Weiter unten werden bevorzugt Kamerasysteme ausführlicher beschrieben.

Eine Oberflächenbild-Erstellungseinheit, ausgelegt zur Erstellung eines dreidimensionalen Oberflächenbildes aus diesen Aufnahmen, wie es oben bereits beschrieben wurde.

Eine Registrierungseinheit zur zumindest bereichsweisen (also z.B. in den Bereichen der als interessant oder nichtinteressant markierten Strukturen) Registrierung des Oberflächenbilds und des Strukturbilds aufeinander. Dieser Vorgang wurde ebenfalls oben bereits beschrieben.

Eine Überlagerungsbild-Erstellungseinheit ausgelegt zur Erstellung eines Überlagerungsbilds wie ebenfalls oben bereits beschrieben wurde. Das Überlagerungsbild umfasst zumindest einen Teil des registrierten Strukturbilds inklusive registrierter Strukturdaten in Form einer Überlagerung mit dem registrierten Oberflächenbild oder der realen Ansicht des entsprechenden Teils des realen Körperbereichs.

Die Steuereinrichtung ist dabei dazu ausgelegt, das medizintechnische bildgebende System dermaßen zu steuern, dass das medizintechnische bildgebende System Bereiche des Patienten basierend auf der Anordnung der registrierten Strukturdaten im Überlagerungsbild aufnimmt. Dazu wird auf die vorangehende Beschreibung zu den interessierenden und nichtinteressierenden Strukturen verwiesen.

Ein erfindungsgemäßes medizintechnisches bildgebendes System umfasst eine solche erfindungsgemäße Steuereinrichtung.

Ein Großteil der zuvor genannten Komponenten des Systems können ganz oder teilweise in Form von Softwaremodulen in einem Prozessor einer entsprechenden Steuer- oder Recheneinrichtung realisiert werden. Eine weitgehend softwaremäßige Realisierung hat den Vorteil, dass auch schon bisher verwendete Infrastruktur auf einfache Weise durch ein Software-Update nachgerüstet werden kann, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Steuereinrichtung eines medizintechnischen bildgebenden Systems ladbar ist, mit Programmabschnitten, um alle Schritte des erfindungsgemäßen Verfahrens auszuführen, wenn das Programm in der Steuereinrichtung ausgeführt wird. Ein solches Computerprogrammprodukt kann neben dem Computerprogramm gegebenenfalls zusätzliche Bestandteile wie z. B. eine Dokumentation bzw. zusätzliche Komponenten auch Hardware-Komponenten, wie z.B. Hardware-Schlüssel (Dongles etc.) zur Nutzung der Software, umfassen.

Bevorzugt ist auch ein Computerlesbares Medium, auf welchem von einer Rechnereinheit einlesbare und ausführbare Programmabschnitte gespeichert sind, um alle Schritte des erfindungsgemäßen Verfahrens auszuführen, wenn die Programmabschnitte von der Rechnereinheit ausgeführt werden.

Weitere, besonders vorteilhafte Ausgestaltungen und Weiterbildungen der Erfindung ergeben sich aus den abhängigen Ansprüchen sowie der nachfolgenden Beschreibung. Dabei können die Merkmale zu Ausführungsformen einer Kategorie auch zur Charakterisierung von Ausführungsformen einer anderen Kategorie dienen. Beispielsweise kann die erfindungsgemäße Steuereinrichtung auch analog zu den abhängigen Verfahrensansprüchen oder Beschreibungsteilen weitergebildet sein, wobei Entsprechendes umgekehrt auch für die Verfahrensansprüche gilt. Es können insbesondere auch einzelne Merkmale verschiedener Ausführungsbeispiele bzw. Varianten zu neuen Ausführungsbeispielen bzw. Varianten kombiniert werden.

Bevorzugt umfasst das Strukturbild eine medizintechnische Bildaufnahme des Körperbereichs des Patienten oder eines anderen Patienten. Die Oberflächendaten und/oder die Strukturdaten sind dabei bevorzugt Bilder von einem realen Körperbereich.

Ebenfalls bevorzugt, alternativ oder ergänzend umfasst das Strukturbild Bilddaten eines Körperbereichs eines künstlich erstellten Avatars. Dabei sind bevorzugt sowohl die Oberflächendaten als auch die inneren Strukturen des Avatars simuliert bzw. berechnet und entsprechen denen einem realen Menschen oder einer statistischen Mittelung über eine Menschengruppe.

Ein Avatar kann dabei ein virtuelles Körpermodell darstellen, z.B. in Form einer Geometrie und organ-basierten Materialeigenschaften. Ein Avatar kann aber auch statistische Modellparameter beinhalten, wie z.B. Formvariationen, Materialeigenschafts-Variationen oder entsprechende Variationen, die z.B. über eine Menschengruppe gewonnen werden.

Bevorzugt wird das dreidimensionale Oberflächenbild wie oben erwähnt mittels eines Tiefeninformationssensors, z.B. einer 3D-Kamera, aufgenommen und umfasst dreidimensionale Bildinformationen über die Körperoberfläche des Patienten zumindest betreffend den vorbestimmten Körperbereich. Vorzugsweise umfasst das Oberflächenbild zusätzlich ein Patientenbild aus zweidimensionalen Grauwert- oder Farbdaten. Damit kann ein Foto- oder Filmbild auf die dreidimensionale Oberfläche projiziert werden, was eine spätere Registrierung einfacher macht, da bei der Registrierung zusätzlich zu den 3D-Daten auch Körpermerkmale verwendet werden können, die durch ihre Farbgebung hervorstechen, z.B. die Augen.

Bevorzugt wird vor der Registrierung das Strukturbild automatisch entsprechend des Oberflächenbildes ausgerichtet und positioniert oder es wird eine entsprechende Transformationsmatrix erzeugt. Dies hat den Vorteil, dass das Strukturbild, z.B. ein Avatar bzw. das 3D-Bild der Voruntersuchung und das Oberflächenbild des Patienten bei der nachfolgenden Registrierung die gleiche Ausrichtung haben und entsprechende Körperareale wie z.B. Bauch, Brust oder Schultern leicht aufeinander registriert werden können. Dieser Vorgang muss nicht zwingend während der Registrierung bzw. mit Mitteln der grafischen Bildregistrierung erfolgen, sondern einfach durch Ausrichtung des Koordinatensystems des Strukturbildes gemäß dem Koordinatensystem des medizintechnischen bildgebenden Systems.

Bevorzugt wird eine grafische (Koordinaten-)Transformation, welche zur Registrierung auf die äußeren Konturen des Strukturbilds angewandt wird, auch entsprechend auf die Strukturdaten des Strukturbilds angewandt. Dies hat den Vorteil, dass die inneren Strukturen des Strukturbildes, welche z.B. Organe des Patienten wiedergeben, auch unabhängig von den Oberflächendaten behandelt werden können. Beispielsweise würden bei einer Streckung der Oberfläche um X% dort auch die inneren Strukturen um X% gestreckt.

Bevorzugt wird zur Ergänzung der Registrierung mittels des bildgebenden medizintechnischen Systems zusätzlich ein Patientenstrukturbild aufgenommen. Dieses weist sowohl innere Strukturen als auch Informationen zur Oberfläche des Patienten auf. Beispielsweise könnte ein "Scout-Scan" (eine übliche Übersichtsaufnahme) für ein Patientenstrukturbild verwendet werden. Dabei entspricht die äußere Kontur des Patienten im Patientenstrukturbild bevorzugt derjenigen im Oberflächenbild, d.h. der Patient sollte sich zwischen der Aufnahme des Oberflächenbildes und des Patientenstrukturbildes möglichst nicht bewegt haben. Insbesondere erfolgt die Aufnahme des Patientenstrukturbildes zusammen mit dem Oberflächenbild, was die Gefahr einer Bewegung des Patienten und einer damit verbundenen Inkongruenz zwischen den beiden Aufnahmen minimiert.

Das Patientenstrukturbild wird vorzugsweise bezüglich seiner Informationen zur Oberfläche des Patienten mit dem Oberflächenbild registriert. Zusätzlich werden in diesem Sinne die inneren Strukturdaten des Strukturbildes mit entsprechenden inneren Strukturen des Patientenstrukturbildes registriert. Die Registrierung kann diesbezüglich z.B. mit Minimierung einer Kostenfunktion erreicht werden, welche die Abstände oder die Abstandsquadrate vorher festgelegter, miteinander übereinstimmender Punkte in den verschiedenen Bildern zueinander beinhaltet.

Beispielsweise erfolgt zunächst ein Scout-Scan und eine Aufnahme des Patienten mit einer 3D-Kamera, daraufhin erfolgt eine Registrierung von Scout-Scan, einem Avatar und der Bildaufnahme der 3D-Kamera. Die Organe des Avatars werden dabei mit Organen des Scout-Scans registriert.

Bevorzugt wird das Überlagerungsbild mit einer Anzeigevorrichtung angezeigt. Dies erlaubt eine Überprüfung der Aufnahme und weitere Möglichkeiten, die im Folgenden genauer ausgeführt werden. Eine bevorzugte Steuereinrichtung weist dementsprechend zusätzlich eine Schnittstelle für eine solche Anzeigevorrichtung und/oder eine Anzeigevorrichtung auf. Die Anzeigevorrichtung ist bevorzugt eine Virtual Reality (VR-) Anzeigevorrichtung oder eine Augmented Reality (AR-) Anzeigevorrichtung, z.B. eine VR-Brille oder AR-Brille.

Die Anzeige des Überlagerungsbildes kann für die Steuerung an sich sehr vorteilhaft sein, insbesondere im funktionellen Zusammenhang mit einer Erfassungseinheit, die dazu ausgebildet ist, weitere Steuerdaten eines Benutzers aufzunehmen. Die Erfassungseinheit kann ein einfaches Bedienelement sein, z.B. eine Tastatur oder eine Maus, bevorzugt ist diese Erfassungseinheit als Gestikerkennungseinheit ausgestaltet oder mit einer Gestikerkennungseinheit funktionell verbunden, die in der Lage ist, vorgegebene Gesten des Benutzers zu erkennen und in Steuerbefehle zu übersetzen. Die bevorzugte Steuereinrichtung umfasst in diesem Falle eine Anzeigevorrichtung und zusätzlich eine Erfassungseinheit. Hierzu können beliebige bekannte Verfahren und Vorrichtungen bzw. Software zur Gestikerkennung eingesetzt werden.

Die durch einen Benutzer mittels der Erfassungseinheit eingegebenen Steuerdaten sind bevorzugt dazu ausgelegt, die Steuerbefehle für die Aufnahme von Bereichen und/oder von Schichten zu ändern oder zu ergänzen. Zusätzlich werden bevorzugt Steuerparameter in dem Überlagerungsbild in geeigneter Form visualisiert werden. Visualisierungen der Steuerparameter können beispielsweise Anzeigen zum aufzunehmenden Bereich sein, z.B. kann dieser Bereich im Überlagerungsbild besonders gekennzeichnet werden, oder Anzeigen zu Schichten, z.B. können die einzelnen Schichten dazu im Überlagerungsbild (ggf. farbig) eingeblendet werden. Die Steuerdaten, die durch den Benutzer über die Erfassungseinheit geändert oder ergänzt werden, können z. B. auf diese Weise bevorzugt in einem neu erstellten Überlagerungsbild angezeigt werden. Wenn der Benutzer z.B. den aufzunehmenden Bereich verschiebt, dann sollte er diese Verschiebung (ggf. in Echtzeit) in einem neuen Überlagerungsbild angezeigt bekommen. Bevorzugt ist auch, dass der Benutzer Schichten bzw. Schichtstapel anders orientieren kann oder den Schichtabstand ändern kann. Auch dies sollte bevorzugt in Form eines neuen Überlagerungsbildes angezeigt werden.

Bevorzugt wird zusätzlich die Position und Orientierung der Anzeigevorrichtung, und damit auch des Betrachters, ermittelt, insbesondere relativ zum Kamerasystem. Dies hat den Vorteil, dass das Überlagerungsbild so berechnet werden kann, dass es aus der Position und Orientierung des Beobachters aus gesehen dargestellt wird. Das Überlagerungsbild wird dabei bevorzugt so in der Anzeigevorrichtung angezeigt, dass der Blickwinkel, aus dem das Überlagerungsbild angezeigt wird, dem der Anzeigevorrichtung auf den Patienten entspricht. Bevorzugt wird dazu das Koordinatensystem der Anzeigevorrichtung, z.B. eines AR- oder VR-Headsets, mit dem Koordinatensystem des Kamerasystems oder des CT registriert. Zusätzlich können Markierungspunkte in dem CT zu dieser Registrierung verwendet werden, was die Genauigkeit verbessert, insbesondere wenn die Anzeigevorrichtung ebenfalls Kameras aufweist, die diese Markierungspunkte aufnehmen.

Bevorzugt werden von vorgegebenen Daten automatisch generierte und/oder durch einen Nutzer manuell erstellte Marker, z.B. für Einschnitte, Einstiche oder andere Penetrationen, und/oder Navigationsdaten und/oder Hilfsdaten, z.B. Label, Verlauf von kritischen Körperbereichen zusätzlich in dem Überlagerungsbild dargestellt. Dies hat insbesondere für einen Befunder oder Operateur, der das Überlagerungsbild mit einer Anzeigevorrichtung betrachtet, den Vorteil, dass er seine nächsten Schritte besser planen kann. Bevorzugt erfolgt die Steuerung des medizintechnischen bildgebenden Systems dermaßen, dass das medizintechnische bildgebende System Bereiche des Patienten basierend auf der Anordnung der registrierten Strukturdaten und einem Marker oder mehreren Markern im Überlagerungsbild aufnimmt.

Bevorzugt werden Bilddaten zu einem virtuellen medizinischen Objekt, z.B. Nadel oder Endoskop in dem Überlagerungsbild dargestellt. Dabei kann vorzugsweise die Orientierung und/oder Position des medizinischen Objekts verändert werden, z.B. verschoben, gezoomt bzw. gedreht. Dies hat den Vorteil, dass virtuell ein optimales Vorgehen bei einer Operation simuliert werden kann.

Bevorzugt kann auch die Position und/oder Orientierung des Überlagerungsbilds durch den Benutzer aktiv verändert werden, z.B. verschoben, vergrößert, verkleinert und/oder gedreht. Dies hat z.B. den Vorteil, dass ein Blick entlang einer einzuführenden Nadel optimal dargestellt werden kann. Eine diesbezügliche Veränderung ist im Rahmen einer VR-Darstellung durch eine einfache Berechnung möglich. Bei einer AR-Darstellung liefert die Kamera ständig Oberflächenbilder und es werden ständig neue Überlagerungsbilder mit dem Verfahren generiert, so dass der Betrachter die Blickrichtung durch Bewegung verändern kann.

Bevorzugt wird das Oberflächenbild aus Bildern erstellt, die mittels einer 3D-Kamera oder mittels einer Anzahl von zur Aufnahme eines dreidimensionalen Bildes verschalteten 2D-Kameras aufgenommen werden. Dabei ist bevorzugt mindestens eine Kamera in einer vorangehend beschriebenen Anzeigevorrichtung integriert und/oder im bildgebenden medizinischen System positioniert.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine Zusammenstellung eines Strukturbildes in Form eines Avatars,
Figur 2 eine schematische Darstellung eines bevorzugten Verfahrensablaufs,
Figur 3 ein bevorzugtes Überlagerungsbild,
Figur 4 eine schematische Darstellung einer bevorzugten Ausführungsform eines erfindungsgemäßen bildgebenden Systems und dessen Funktionsweise.

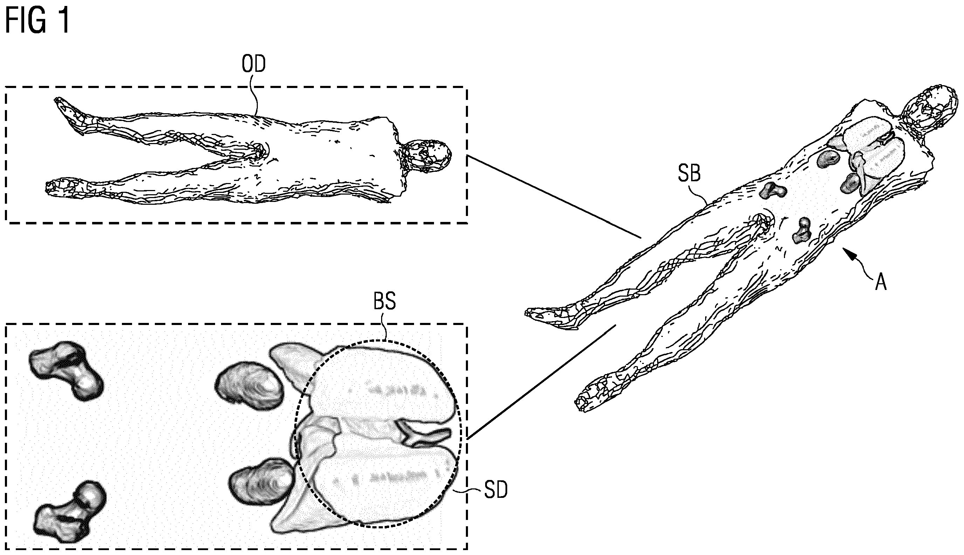

Figur 1 zeigt schematisch eine mögliche Zusammenstellung eines Strukturbildes SB in Form eines Avatars A aus Oberflächendaten OD und Strukturdaten SD, BS.

Als Oberflächendaten OD werden Daten der Oberfläche einer Person P verwendet. Diese können im einfachsten Falle aus einer realen 3D-Aufnahme einer Person gebildet werden. In der Praxis ist es jedoch sinnvoll, wenn die Oberflächendaten OD aus den Oberflächen mehrerer Personen als Mittelwertdaten berechnet werden, um den Avatar A auf möglichst viele Personen anwenden zu können. Denkbar ist auch, die Oberflächendaten OD aus grafischen Modellen zu berechnen. In diesem Falle liegt der Schwerpunkt auf inneren Organen des Oberkörpers, so dass eine Darstellung der Arme entfallen kann.

Die Strukturdaten SD sind hier Daten von inneren Organen eines Menschen. Sie können aber auch Knochen, Gefäße, Nerven oder beliebige andere Strukturen enthalten. Auch die Strukturdaten SD können im einfachsten Falle aus einer realen Aufnahme einer Person P, z.B. einer CT-Aufnahme gebildet werden. In der Praxis ist es jedoch auch hier sinnvoll, wenn die Strukturdaten SD aus Aufnahmen mehrerer Personen als Mittelwertdaten berechnet werden, um den Avatar A auf möglichst viele unterschiedliche Personen anwenden zu können. Denkbar ist ebenso, die Strukturdaten SD aus grafischen Modellen zu berechnen.

In diesem Beispiel sind die Lungen als besondere Strukturen BS markiert, was durch einen gestrichelt umgebenen Bereich dargestellt wird. Diese besonderen Strukturen können beispielsweise als interessierende Strukturen definiert sein, was zu einer gezielten Aufnahme der Lunge führen würde. Es kann aber auch sein, dass die besonderen Strukturen als nicht-interessierende Strukturen definiert sind, was zu einer gezielten Vermeidung einer Aufnahme der Lunge führen würde.

Die Oberflächendaten OD und Strukturdaten SD können bereits bei der Erstellung des Avatars A in einem einzigen Datensatz vorliegen. In dem hier gezeigten Fall werden die Strukturdaten SD (und damit auch BS) mit den Oberflächendaten registriert, z.B. indem beide Datensätze in vergleichbaren Koordinatensystemen erstellt worden sind und entsprechend der Koordinaten aufeinander abgebildet werden.

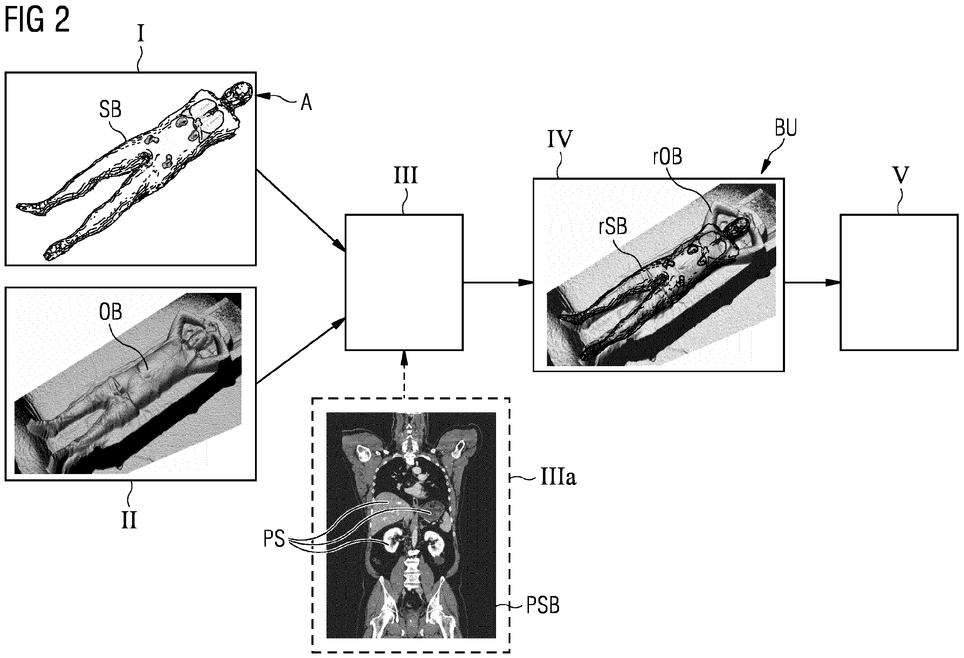

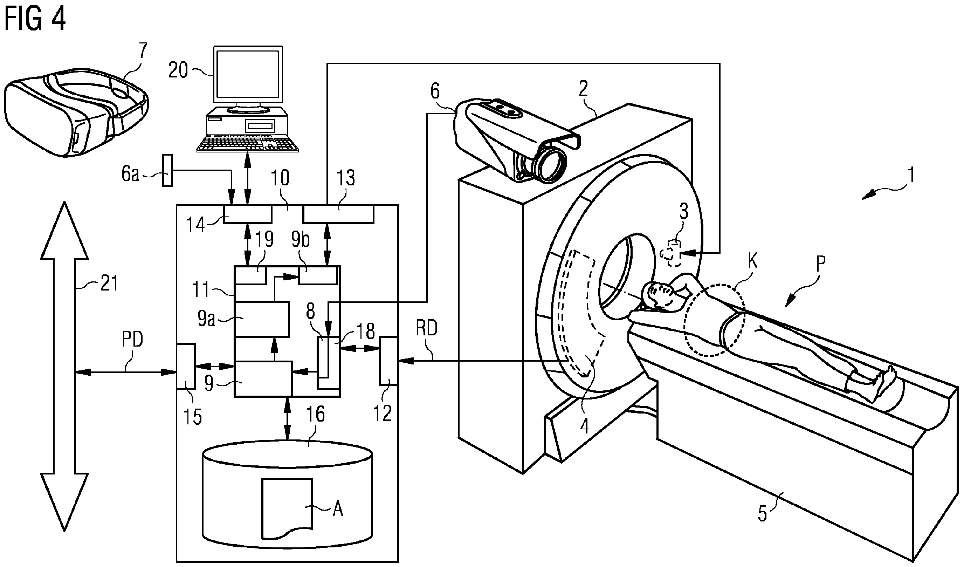

Figur 2 zeigt eine schematische Darstellung eines bevorzugten Verfahrensablaufs zur Steuerung eines medizintechnischen bildgebenden Systems 1, wie es z.B. in Figur 4 dargestellt ist, in Form eines Blockdiagramms.

In Schritt I wird ein dreidimensionales Strukturbild SB bereitgestellt, welches hier ein Avatar A ist. Es könnte hier jedoch auch eine vorher erstellte CT-Aufnahme des Patienten verwendet werden, wenn eine solche bereits vorliegt. Wie vorangehend zu Figur 1 bereits beschrieben wurde, umfasst das Strukturbild SB Oberflächendaten OD zu äußeren Konturen sowie Strukturdaten SD zu inneren Strukturen eines Körperbereichs K eines Patienten P.

In Schritt II erfolgt eine Aufnahme des Körperbereichs K des Patienten P mit einem Kamerasystem 6, während sich der Patient P in dem bildgebenden medizinischen System 1 befindet (s. dazu auch Fig. 4), und die Erstellung eines dreidimensionalen Oberflächenbildes OB aus diesen Aufnahmen.

In Schritt 3 erfolgt eine Registrierung des Oberflächenbilds OB und der Oberflächendaten OD des Strukturbilds SB aufeinander. Dabei werden auch die Strukturdaten SD zusammen mit dem Strukturbild SB registriert, d.h. alle grafischen Transformationen, die zur Registrierung mit dem Strukturbild vorgenommen werden müssen, werden entsprechend auch auf die Strukturdaten SD angewandt.

In einem optionalen Schritt IIIa kann eine vorher erstellte Aufnahme der realen Strukturen des Patienten, z.B. ein Scout-scan, als Patientenstrukturbild PSB verwendet werden, um die Strukturdaten SD unabhängig von den Oberflächendaten OD auf die realen inneren Strukturen PS des Patienten zu registrieren. Dies führt zu einer verbesserten Abbildung der Strukturdaten SD im Überlagerungsbild UB.

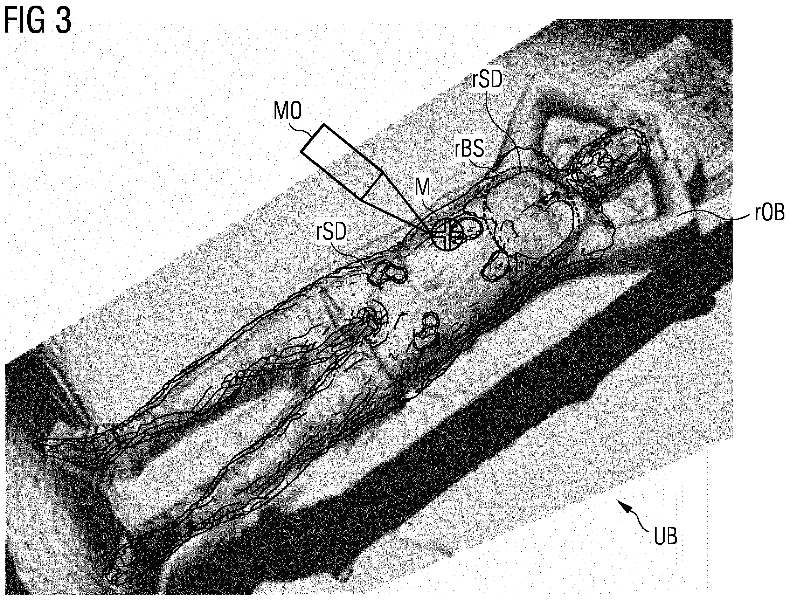

In Schritt IV erfolgt eine Erstellung eines Überlagerungsbilds UB, welches in Figur 3 etwas genauer dargestellt ist. Das Überlagerungsbild UB umfasst zumindest einen Teil des registrierten Strukturbilds rSB in Form einer Überlagerung mit dem registrierten Oberflächenbild rOB.

In Schritt V erfolgt eine Steuerung des medizintechnischen bildgebenden Systems 1 dermaßen, dass das medizintechnische bildgebende System 1 Bereiche des Patienten P basierend auf den registrierten Strukturdaten rSD im Überlagerungsbild aufnimmt. Da das Überlagerungsbild die reale Körperansicht des Patienten wiedergibt, kann die Steuerung einfach im Koordinatensystem des bildgebenden Systems erfolgen.

Figur 3 zeigt perspektivisch ein bevorzugtes Überlagerungsbild UB aus einem registrierten Oberflächenbild rOB, einem registrierten Strukturbild rSB enthaltend markierte besondere Strukturen rBS, einer virtuellen Markierung M und einem virtuellen medizinischen Objekt MO.

Figur 4 zeigt eine schematische Darstellung einer bevorzugten Steuereinrichtung 10 zur Steuerung eines medizintechnischen bildgebenden Systems 1, das hier ein Computertomographiesystem 1 ist. Bei der Steuereinrichtung 10 sind nur diejenigen Komponenten dargestellt, die für die Erläuterung der Erfindung wesentlich oder hilfreich sind.

Das Computertomographiesystem 1 weist in üblicher Weise einen Scanner 2 mit einer Gantry auf, in der eine Röntgenquelle 3 rotiert, die jeweils einen Patienten P durchstrahlt, welcher mittels einer Liege 5 in einen Messraum der Gantry hineingeschoben wird, so dass die Strahlung auf einen der Röntgenquelle 3 jeweils gegenüberliegenden Detektor 4 trifft. Es soll dabei eine Aufnahme eines bestimmten Körperbereichs K vorgenommen werden.

Eine Kernkomponente der Steuereinrichtung 10 ist hier ein Prozessor 11, auf dem verschiedene Komponenten in Form von Softwaremodulen realisiert sind. Die Steuereinrichtung 10 weist weiterhin eine Terminalschnittstelle 14 auf, an die ein Terminal 20 angeschlossen ist, über das ein Bediener die Steuereinrichtung 10 und somit das Computertomographiesystem 1 bedienen kann. Eine weitere Schnittstelle 15 ist eine Netzwerkschnittstelle zum Anschluss an einen Datenbus 21, um so eine Verbindung zu einem Radiologischen Informationssystem ("RIS") bzw. oder einem Bildarchivierungssystem ("PACS" für "Picture Archiving and Communication System") herzustellen. Über diese Schnittstelle 15 und diesen Datenbus 21 werden in diesem Beispiel auch die Daten des Überlagerungsbildes an eine Anzeigevorrichtung 7 gesendet, die hier eine VR- bzw. AR-Brille 7 ist.

Über eine Steuerschnittstelle 13 kann von der Steuereinrichtung 10 der Scanner 2 angesteuert werden, d.h. es werden z.B. die Rotationsgeschwindigkeit der Gantry, die Verschiebung der Liege 5 und die Röntgenquelle 3 selbst gesteuert. Auf diese Weise kann ein klar definierter Bereich aufgenommen werden. Dabei kann insbesondere, wie oben bereits ausgeführt wurde, eine Steuerung der Schichtorientierung, des Schichtabstands und/oder der Anzahl der Schichten erfolgen.

Über eine Akquisitionsschnittstelle 12 werden die Rohdaten RD aus dem Detektor 4 ausgelesen. Die Rohdaten werden mittels einer Bildrekonstruktionseinheit 18 entsprechend rekonstruiert.

Weiterhin weist die Steuereinrichtung 10 eine Speichereinheit 16 auf, die Daten zur Steuerung enthalten kann. In dieser Speichereinheit 16 sind in diesem Beispiel auch die Daten zu potentiellen Avataren A gespeichert.

Eine weitere Komponente auf dem Prozessor 11 ist eine Oberflächenbild-Erstellungseinheit 8, welche dazu ausgelegt ist, aus den Bildern eines Kamerasystems 6 von dem Patienten P, zumindest des Körperbereichs K ein dreidimensionales Oberflächenbild OB des Patienten zu erstellen. In diesem Beispiel ist die Oberflächenbild-Erstellungseinheit 8 Teil der Bildrekonstruktionseinheit 18, bzw. die Funktionalität der Bildrekonstruktionseinheit 18 wurde um die Funktionalität der Oberflächenbild-Erstellungseinheit 8 ergänzt. Das Kamerasystem ist hier zur besseren Sichtbarkeit auf dem Scanner dargestellt. In der Praxis wäre es vorteilhaft, wenn es sich im Inneren des Scanners befinden würde, um den Patienten ständig, auch während einer CT-Aufnahme, aufzunehmen.

In dem dargestellten Beispiel ist die Oberflächenbild-Erstellungseinheit 8 auch dazu ausgelegt, Rohdaten des Detektors 1 zu empfangen und ggf. auch zu rekonstruieren. Auf diese Weise kann die Oberflächenbild-Erstellungseinheit 8 zusätzlich ein Patientenstrukturbild PSB, z.B. aus einem Scout-Scan, der Steuereinrichtung zur Verfügung stellen, wie es als Option in Figur 2 gezeigt ist.

Eine weitere Komponente auf dem Prozessor 11 ist eine Registrierungseinheit 9. Diese registriert das Oberflächenbild OB und die Oberflächendaten OD des Avatars A und ggf. auch die Strukturdaten SD des Avatars mit den Patientenstrukturdaten PD aufeinander.

Eine weitere Komponente auf dem Prozessor 11 ist eine Überlagerungsbild-Erstellungseinheit 9a, die ein Überlagerungsbild aus dem registrierten Oberflächenbild rOB und dem registrierten Strukturbild rSB zusammen mit den registrierten Strukturdaten rSD erzeugt.

Basierend auf dem Überlagerungsbild bzw. des in diesem enthaltenen registrierten Strukturbild rSB zusammen mit den registrierten Strukturdaten rSD ist es möglich, den Computertomographen 1 dermaßen zu steuern, dass spezielle Bereiche des Patienten P gezielt aufgenommen oder gezielt nicht aufgenommen werden. Dies kann beispielsweise durch eine Erkennungseinheit 9b realisiert werden, die die registrierten Strukturdaten rSD, insbesondere markierte besondere Strukturdaten rBS, erkennt, deren Koordinaten mit den realen Koordinaten des Patienten abgleicht, was einfach ist, da das Überlagerungsbild die realen Koordinaten des Patienten wiedergibt, und bei der Steuerung des Geräts stets den entsprechenden Bereich in dem Falle, dass die besonderen Strukturdaten interessierende Bereiche darstellen, aufnimmt und in dem Falle, dass die besonderen Strukturdaten nichtinteressierende Bereiche darstellen, nicht aufnimmt.

Als bevorzugte Ergänzung umfasst die Steuereinrichtung 10 zusätzlich eine Erfassungseinheit 19, die dazu ausgebildet ist, weitere Steuerdaten durch einen Benutzer aufzunehmen. Hier ist die Erfassungseinheit 19 als Gestikerkennungseinheit ausgestaltet, die in der Lage ist, vorgegebene Gesten des Benutzers zu erkennen und in Steuerbefehle zu übersetzen. Dazu ist zusätzlich eine Kamera 6a vorhanden. Die Erfassungseinheit 19 erhält hier Daten über die Schnittstelle 14, die wiederum Daten von der Kamera 6a erhält. Zusätzlich kann die Erfassungseinheit 19 auch Daten von dem Terminal 20 erhalten. Z. B. könnte der Benutzer so in der VR- bzw. AR-Umgebung (d.h. in der "Realität", die er in einem mittels der VR- bzw. AR-Brille 7 angezeigte Überlagerungsbild sieht) in Echtzeit visualisierte Steuerparameter, wie z.B. Schichtanordnungen etc., für eine nachfolgenden Aufnahme verändern, neu hinzunehmen oder löschen. Beispielsweise könnte er einen angezeigten Schichtstapel mit den Händen zusammenschieben oder auseinanderziehen (um die Schichtabstände zu verringern oder zu erweitern), den Stapel im Raum drehen oder auf andere Weise umpositionieren oder neue Schichten hinzufügen oder Schichten löschen.

Es wird abschließend noch einmal darauf hingewiesen, dass es sich bei den vorhergehend detailliert beschriebenen Verfahren sowie bei der dargestellten Vorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriff "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Steuerung eines medizintechnischen bildgebenden Systems (1), umfassend die Schritte:
- Bereitstellung eines dreidimensionalen Strukturbilds (SB) umfassend sowohl Oberflächendaten (OD) zu äußeren Konturen sowie Strukturdaten (SD) zu inneren Strukturen eines Körperbereichs (K),
- Aufnahme des Körperbereichs (K) eines Patienten (P) mit einem Kamerasystem (6), während sich der Patient (P) in oder auf dem bildgebenden medizinischen System (1) befindet, und Erstellung eines dreidimensionalen Oberflächenbildes (OB) aus diesen Aufnahmen,
- zumindest bereichsweise Registrierung des Oberflächenbilds (OB) und des Strukturbilds (SB) aufeinander,
**dadurch gekennzeichnet, dass** das Verfahren folgende Schritte umfasst:
- Erstellung eines Überlagerungsbilds (UB), wobei das Überlagerungsbild (UB) zumindest einen Teil des registrierten Strukturbilds (rSB) inklusive der registrierten Strukturdaten (rSD) in Form einer Überlagerung mit dem registrierten Oberflächenbild (rOB) und/oder der realen Ansicht des entsprechenden Teils des realen Körperbereichs (K) umfasst,
- Steuerung des medizintechnischen bildgebenden Systems (1) dermaßen, dass das medizintechnische bildgebende System (1) Bereiche des Patienten (P) basierend auf der Anordnung der registrierten Strukturdaten (rSD) im Überlagerungsbild aufnimmt.

2. Verfahren nach Anspruch 1, wobei das Strukturbild (SB)
- auf einer medizintechnische Bildaufnahme des Körperbereichs (K) dieses Patienten (P) oder zumindest eines anderen Patienten (P) basiert,
oder
- Bilddaten eines Körperbereichs (K) eines künstlich erstellten Avatars (A) umfasst.

3. Verfahren nach einem der vorangehenden Ansprüche, wobei vor der Registrierung das Strukturbild (SB) automatisch entsprechend dem Oberflächenbild (OB) ausgerichtet und positioniert wird oder eine entsprechende Transformationsmatrix erzeugt wird.

4. Verfahren nach einem der vorangehenden Ansprüche, wobei eine grafische Transformation, welche zur Registrierung auf die äußeren Konturen (OD) des Strukturbilds (SB) angewandt wird, entsprechend auf die Strukturdaten (SD) des Strukturbilds (SB) angewandt wird.

5. Verfahren nach einem der vorangehenden Ansprüche, wobei zur Ergänzung der Registrierung mittels des bildgebenden medizintechnischen Systems (1) zusätzlich ein Patientenstrukturbild (PSB) aufgenommen wird, welches innere Strukturen (PS) und Informationen zur Oberfläche des Patienten (P) aufweist, und das Patientenstrukturbild (PSB) bezüglich seiner Informationen zur Oberfläche des Patienten (P) mit dem Oberflächenbild (OB) registriert wird und zusätzlich die inneren Strukturdaten (SD) des Strukturbildes (SB) mit entsprechenden inneren Strukturen (PS) des Patientenstrukturbildes (PSB) registriert werden.

6. Verfahren nach einem der vorangehenden Ansprüche, wobei das Überlagerungsbild mit einer Anzeigevorrichtung (7) angezeigt wird, insbesondere einer Virtual Reality Anzeigevorrichtung oder einer Augmented Reality Anzeigevorrichtung, und bevorzugt zusätzlich die Position und Orientierung der Anzeigevorrichtung (7) ermittelt wird, und
wobei das Überlagerungsbild (UB) bevorzugt so angezeigt wird, dass der Blickwinkel, aus dem das Überlagerungsbild (UB) angezeigt wird, dem der Anzeigevorrichtung (7) auf den Patienten entspricht.

7. Verfahren nach einem der vorangehenden Ansprüche, wobei basierend auf vorgegebenen Daten automatisch generierte oder manuell erzeugte Marker (M), und/oder Navigationsdaten und/oder Hilfsdaten zusätzlich in dem Überlagerungsbild (UB) dargestellt werden, wobei bevorzugt die Steuerung des medizintechnischen bildgebenden Systems (1) dermaßen erfolgt, dass das medizintechnische bildgebende System (1) Bereiche des Patienten (P) basierend auf der Anordnung der registrierten Strukturdaten (rSD) und der Marker (M) im Überlagerungsbild aufnimmt.

8. Verfahren nach einem der vorangehenden Ansprüche, wobei Bilddaten zu einem virtuellen medizinischen Objekt (MO) in dem Überlagerungsbild (UB) dargestellt werden, wobei die Orientierung und/oder Position des medizinischen Objekts (MO) verändert werden kann,
wobei bevorzugt auch die Position und/oder Orientierung des Überlagerungsbilds (UB) durch den Benutzer aktiv verändert werden kann.

9. Verfahren nach einem der vorangehenden Ansprüche, wobei das Oberflächenbild (OB) aus Bildern erstellt wird, die mittels einer 3D-Kamera (15) oder mittels einer Anzahl von zur Aufnahme eines dreidimensionalen Bildes verschalteten 2D-Kameras (15) aufgenommen werden, wobei bevorzugt mindestens eine Kamera (15) in der Anzeigevorrichtung (7) integriert ist und/oder im bildgebenden medizinischen System (1) positioniert ist.

10. Steuereinrichtung (10) zur Steuerung eines medizintechnischen bildgebenden Systems (1) umfassend
- eine Strukturbildschnittstelle (14) zur Bereitstellung eines dreidimensionalen Strukturbildes (SB) umfassend sowohl Oberflächendaten (OD) zu äußeren Konturen sowie Strukturdaten (SD) zu inneren Strukturen (BS) eines Körperbereichs (1),
- ein Kamerasystem (6) zur Aufnahme zumindest des Körperbereichs (K) eines Patienten (P) in einem bildgebenden medizinischen System (1),
- eine Oberflächenbild-Erstellungseinheit (8), ausgelegt zur Erstellung eines dreidimensionalen Oberflächenbildes (OB) aus diesen Aufnahmen,
- eine Registrierungseinheit (9) zur zumindest bereichsweisen Registrierung des Oberflächenbilds (OB) und des Strukturbilds (SB) aufeinander, **dadurch gekennzeichnet,**
**dass** die Steuerungseinheit folgende Einheiten umfasst:
- eine Überlagerungsbild-Erstellungseinheit (9a) ausgelegt zur Erstellung eines Überlagerungsbilds (UB), wobei das Überlagerungsbild (UB) zumindest einen Teil des registrierten Strukturbilds (rSB) inklusive der registrierten Strukturdaten (rSD) in Form einer Überlagerung mit dem registrierten Oberflächenbild (rOB) oder der realen Ansicht des entsprechenden Teils des realen Körperbereichs (K) umfasst,
wobei die Steuereinrichtung (10) dazu ausgelegt ist, das medizintechnische bildgebende System (1) dermaßen zu steuern, dass das medizintechnische bildgebende System (1) Bereiche des Patienten (P) basierend auf der Anordnung der registrierten Strukturdaten (rSD) im Überlagerungsbild aufnimmt.

11. Steuereinrichtung nach Anspruch 10, welche zusätzlich eine Schnittstelle (15) für eine Anzeigevorrichtung (7) und/oder eine Anzeigevorrichtung (7) aufweist, die zur Anzeige des Überlagerungsbildes (UB) ausgestaltet ist.

12. Medizintechnisches bildgebendes System umfassend eine Steuereinrichtung (10) nach Anspruch 10 oder 11.

13. Computerprogrammprodukt mit einem Computerprogramm, welches direkt in eine Speichereinrichtung einer Steuereinrichtung (10) ladbar ist, mit Programmabschnitten, um alle Schritte des Verfahrens nach einem der Ansprüche 1 bis 9 auszuführen, wenn das Computerprogramm in der Steuereinrichtung (10) ausgeführt wird.

14. Computerlesbares Medium, auf welchem von einer Rechnereinheit einlesbare und ausführbare Programmabschnitte gespeichert sind, um alle Schritte des Verfahrens nach einem der Ansprüche 1 bis 9 auszuführen, wenn die Programmabschnitte von der Rechnereinheit ausgeführt werden.

## Claims

1. Method for controlling a medical imaging system (1), comprising the steps:
- providing a three-dimensional structure image (SB) comprising both surface data (OD) relating to external contours and structure data (SD) relating to internal structures of a body region (K),
- recording the body region (K) of a patient (P) by means of a camera system (6) while the patient (P) is situated in or on the medical imaging system (1), and producing a three-dimensional surface image (OB) from these recordings,
- registering at least locally the surface image (OB) and the structure image (SB) in relation to each other,
**characterised in that** the method comprises the following steps:
- producing an overlay image (UB), wherein the overlay image (UB) comprises at least part of the registered structure image (rSB) including the registered structure data (rSD) in the form of an overlay with the registered surface image (rOB) and/or the real view of the corresponding part of the real body region (K),
- controlling the medical imaging system (1) in such a way that the medical imaging system (1) records regions of the patient (P) on the basis of the arrangement of the registered structure data (rSD) in the overlay image.

2. Method according to claim 1, wherein the structure image (SB)
- is based on a medical image recording of the body region (K) of this patient (P) or at least another patient (P), or
- comprises image data of a body region (K) of an artificially produced avatar (A).

3. Method according to one of the preceding claims, wherein before the registration, the structure image (SB) is automatically aligned and positioned according to the surface image (OB) or a corresponding transformation matrix is generated.

4. Method according to one of the preceding claims, wherein a graphical transformation which is applied to the external contours (OD) of the structure image (SB) for the purpose of registration, is applied to the structure data (SD) of the structure image (SB) correspondingly.

5. Method according to one of the preceding claims, wherein a patient structure image (PSB) which features internal structures (PS) and information relating to the surface of the patient (P) is additionally recorded by means of the medical imaging system (1) in order to supplement the registration, and the patient structure image (PSB) is registered with the surface image (OB) in respect of its information relating to the surface of the patient (P), and the internal structure data (SD) of the structure image (SB) is additionally registered with corresponding internal structures (PS) of the patient structure image (PSB).

6. Method according to one of the preceding claims, wherein the overlay image is displayed by means of a display device (7), in particular a Virtual Reality display device or an Augmented Reality display device, and
the position and orientation of the display device (7) are preferably also determined, and
wherein the overlay image (UB) is preferably displayed in such a way that the viewing angle from which the overlay image (UB) is displayed corresponds to that of the display device (7) onto the patient.

7. Method according to one of the preceding claims, wherein markers (M) that are generated automatically on the basis of specified data or produced manually and/or navigation data and/or help data are also represented in the overlay image (UB), wherein the control of the medical imaging system (1) preferably takes place in such a way that the medical imaging system (1) records regions of the patient (P) on the basis of the arrangement of the registered structure data (rSD) and the markers (M) in the overlay image.

8. Method according to one of the preceding claims, wherein image data relating to a virtual medical object (MO) is represented in the overlay image (UB), wherein the orientation and/or position of the medical object (MO) can be changed, wherein the position and/or orientation of the overlay image (UB) can preferably also be actively changed by the user.

9. Method according to one of the preceding claims, wherein the surface image (OB) is produced from images which are recorded by means of a 3D camera (15) or by means of a number of 2D cameras (15) which are interconnected for the purpose of recording a three-dimensional image, wherein at least one camera (15) is preferably integrated in the display device (7) and/or positioned in the medical imaging system (1).

10. Control device (10) for controlling a medical imaging system (1), comprising
- a structure image interface (14) for providing a three-dimensional structure image (SB) comprising both surface data (OD) relating to external contours and structure data (SD) relating to internal structures (BS) of a body region (1),
- a camera system (6) for recording at least the body region (K) of a patient (P) in a medical imaging system (1),
- a surface image production unit (8) which is designed to produce a three-dimensional surface image (OB) from these recordings,
- a registration unit (9) for at least local registration of the surface image (OB) and the structure image (SB) in relation to each other,
**characterised in that** the controller unit comprises the following units:
- an overlay image production unit (9a) which is designed to produce an overlay image (UB), wherein the overlay image (UB) comprises at least part of the registered structure image (rSB) including the registered structure data (rSD) in the form of an overlay with the registered surface image (rOB) or the real view of the corresponding part of the real body region (K),
wherein the control device (10) is designed to control the medical imaging system (1) in such a way that the medical imaging system (1) records regions of the patient (P) on the basis of the arrangement of the registered structure data (rSD) in the overlay image.

11. Control device according to claim 10, also comprising an interface (15) for a display device (7) and/or a display device (7), which is designed to display the overlay image (UB) .

12. Medical imaging system comprising a control device (10) according to claim 10 or 11.

13. Computer program product comprising a computer program which can be loaded directly into a memory device of a control device (10), having program sections for executing all steps of the method according to one of claims 1 to 9 when the computer program is executed in the control device (10).

14. Computer-readable medium on which are stored program sections that can be read in and executed by a computer unit, in order to execute all steps of the method according to one of claims 1 to 9 when the program sections are executed by the computer unit.

## Revendications

1. Procédé de commande d'un système (1) d'imagerie de la technique médicale, comprenant les stades :
- on se procure une image (SB) de structure en trois dimensions, comprenant à la fois des données (OD) de surface de contours extérieurs, ainsi que des données (SD) de structures internes d'une partie (K) du corps,
- on enregistre la partie (K) du corps d'un patient (P) par un système (6) de caméra, pendant que le patient (P) se trouve dans ou sur le système (1) d'imagerie médicale, et on produit une image (OB) de surface en trois dimensions à partir de ces enregistrements,
- on enregistre, au moins par endroit, l'image (OB) de surface et l'image (SB) de structure l'une sur l'autre,
**caractérisé en ce que** le procédé comprend les stades suivantes :
- on produit une image (UB) de superposition, l'image (UB) de superposition comprenant au moins une partie de l'image (rSB) de structure enregistrée, y compris les données (rSD) de structure enregistrées, sous la forme d'une superposition avec l'image (rOB) de surface enregistrée et/ou la vue réelle de la partie correspondante de la partie (K) réelle du corps,
- on commande le système (1) d'imagerie de la technique médicale de manière à ce que le système (1) d'imagerie de la technique médicale enregistre des parties du patient (P) sur la base de l'agencement des données (rSD) de structure enregistrées dans l'image de superposition.

2. Procédé suivant la revendication 1, dans lequel l'image (SB) de structure
- repose sur un enregistrement d'image de la technique médicale de la partie (K) du corps de ce patient (P) ou au moins d'un autre patient (P),
ou
- comprend des données d'image d'une partie (K) du corps d'un avatar (A) produit artificiellement.

3. Procédé suivant l'une des revendications précédentes, dans lequel, avant l'enregistrement on oriente et on met en position l'image (SB) de structure automatiquement, conformément à l'image (OB) de surface ou on produit une matrice de transformation correspondante.

4. Procédé suivant l'une des revendications précédentes, dans lequel on applique, conformément aux données (SD) de structure de l'image (SB) de structure, une transformation graphique, qui est appliquée pour l'enregistrement aux contours (OD) extérieurs de l'image (SB) de structure.

5. Procédé suivant l'une des revendications précédentes, dans lequel, pour compléter l'enregistrement au moyen du système (1) d'imagerie de la technique médicale, on enregistre supplémentairement une image (PSB) de structure du patient, qui a des structures (PS) internes et des informations sur la surface du patient (P), et on enregistre, avec l'image (OB) de surface, l'image (PSB) de structure du patient en ce qui concerne ses informations sur la surface du patient (P) et on enregistre supplémentairement les données (SD) de structure internes de l'image (SB) de structure avec des structures (PS) internes correspondantes de l'image (PSB) de structure du patient.

6. Procédé suivant l'une des revendications précédentes, dans lequel on affiche l'image de cette superposition par un dispositif (7) d'affichage, notamment un dispositif d'affichage en réalité virtuelle ou un dispositif d'affichage en réalité augmentée, et on détermine de préférence supplémentairement la position et l'orientation du dispositif (7) d'affichage, et
dans lequel on affiche de préférence l'image (UB) de superposition, de manière à ce que l'angle de vue, à partir duquel l'image (UB) de superposition est affichée, corresponde sur le patient à celui du dispositif (7) d'affichage.

7. Procédé suivant l'une des revendications précédentes, dans lequel, sur la base de données données à l'avance, on représente supplémentairement, dans l'image (UB) de superposition, des repères (M) créés automatiquement ou produits manuellement et/ou des données de navigation et/ou des données auxiliaires, dans lequel, de préférence, on effectue la commande du système (1) d'imagerie de la technique médicale, de manière à ce que le système (1) d'imagerie de la technique médicale enregistre des parties du patient (P) sur la base des données (rSD) de structure enregistrées et des repères (M) dans l'image de superposition.

8. Procédé suivant l'une des revendications précédentes, dans lequel on représente des données d'image d'un objet (MO) virtuel de médecine dans l'image (UB) de superposition, l'orientation et/ou la position de l'objet (MO) de médecine pouvant être modifiée,
dans lequel, de préférence, la position et/ou l'orientation de l'image (UB) de superposition peut être modifiée activement par l'utilisateur.

9. Procédé suivant l'une des revendications précédentes, dans lequel on produit l'image (OB) de surface à partir d'images, qui sont enregistrées au moyen d'une caméra (15) en 3D ou au moyen d'un certain nombre de caméras (15) en 2D, connectées pour l'enregistrement d'une image en trois dimensions, dans lequel, de préférence, au moins une caméra (15) est intégrée au dispositif (7) d'affichage et/ou est mise en position dans le système (1) d'imagerie médicale.

10. Dispositif (10) de commande pour commander un système (1) d'imagerie de la technique médicale, comprenant
- une interface (14) d'image de structure pour mettre à disposition une image (SB) de structure en trois dimensions, comprenant à la fois des données (OD) de surface de contours extérieurs, et des données (SD) de structure de structures (BS) internes d'une partie (1) d'un corps,
- un système (6) de caméra d'enregistrement d'au moins la partie (K) du corps d'un patient (P) dans un système (1) d'imagerie médicale,
- une unité (8) de production d'image de surface, conçue pour produire une image (OB) de surface en trois dimensions à partir de ces enregistrements,
- une unité (9) d'enregistrement pour enregistrer, au moins par endroit, l'image (OB) de surface et l'image (SB) de structure l'une sur l'autre,
**caractérisé en ce que** l'unité de commande comprend les unités suivantes :
- une unité (9a) de production d'une image de superposition, conçue pour produire une image (UB) de superposition, l'image (UB) de superposition comprenant au moins une partie de l'image (rSB) de structure enregistrée, y compris les données (rSD) de structure enregistrées, sous la forme d'une superposition, avec l'image (rOB) de surface enregistrée ou la vue réelle de la partie correspondante de la partie (K) réelle du corps,
dans lequel le dispositif (10) de commande est conçu pour commander le système (1) d'imagerie de la technique médicale, de manière à ce que le système (1) d'imagerie de la technique médicale enregistre des parties du patient (P) sur la base de l'agencement des données (rSD) de structure enregistrées dans l'image de superposition.

11. Dispositif de commande suivant la revendication 10, qui a supplémentairement une interface (15) pour un dispositif (7) d'affichage et/ou un dispositif (7) d'affichage, qui est conformé pour afficher l'image (UB) de superposition.

12. Système d'imagerie de la technique médicale, comprenant un dispositif (10) de commande suivant la revendication 10 ou 11.

13. Produit de programme d'ordinateur, ayant un programme d'ordinateur, qui peut être chargé directement dans un dispositif de mémoire d'un dispositif (10) de commande, comprenant des parties de programme pour effectuer tous les stades du procédé suivant l'une des revendications 1 à 9, lorsque le programme d'ordinateur est réalisé dans le dispositif (10) de commande.

14. Support déchiffrable par ordinateur, sur lequel des parties de programme déchiffrables et réalisables par une unité d'ordinateur sont mémorisées pour effectuer tous les stades du procédé suivant l'une des revendications 1 à 9, lorsque les parties de programme sont réalisées par l'unité d'ordinateur.
